# EUROPEAN PATENT APPLICATION

(11) **EP 2 371 816 A1**
(43) Date of publication of application: **05.10.2011**
(21) Application number: 10425108.7
(22) Date of filing: 01.04.2010
(51) Int. Cl.: C07D 211/14, C07D 213/53, C07D 265/30, A61K 31/44, A61K 31/4453, A61K 31/5355, A61P 35/00

(54) **Aminocyclohexenone, their preparation and use as medicament against oxidative stress**

(71) Applicant: ABIOGEN PHARMA S.p.A., 56121 Localita Ospedaletto, Pisa (IT)
(72) Inventor: Napolitano, Elio, 56127 Pisa (IT); Basagni, Simone, 56124 Pisa (IT); Trasciatti, Silvia, 56010 Pisa (IT)
(74) Representative: Cattaneo, Elisabetta

(57) **Abstract**

The present invention concerns amino derivatives of cyclohex-2-enone of formula I : 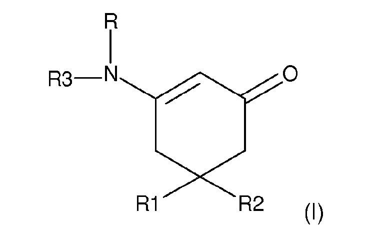 and the salts thereof. Said derivatives have proved to be particularly effective as antioxidants in the treatment of cellular oxidative stress, especially at CNS level.

## Description

### FIELD OF THE INVENTION

The present invention concerns amino derivatives of cyclohex-2-enone of formula: 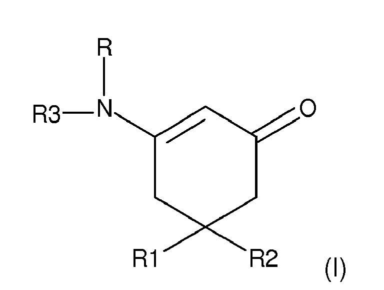 or a salt thereof, wherein R, R1, R2 and R3 are as hereinafter defined. Said derivatives have proved to be particularly effective as antioxidants in the treatment of cellular oxidative stress, especially at CNS level.

### STATE OF THE ART

The concept of "oxidative stress" is known to refer to the hyper-production and diffusion in cells of specific highly reactive substances, characterized by the presence of an oxygen atom with an unpaired electron.

Such free radicals are strongly oxidizing species, which tend to react forming other radicals by removing an electron or a hydrogen atom from other nearby molecules. The net resultant effect is the propagation of the production of unstable and highly reactive molecules.

The presence of free radicals, in the absence of an adequate defense system, can trigger an actual cascade of reactions that are highly damaging to the cell and negatively affect its structural and functional integrity.

This attack on the cell membrane by free radicals results in an altered cell membrane permeability. Further damage to the DNA can occur with consequent triggering of genomic alterations leading to the onset of chromosomal mutations and degenerative processes in the cell. As often observed, under certain conditions, free radicals are able to injure cells and tissues, resulting in the onset of chronic-degenerative, cardiovascular and chronic inflammatory alterations which are responsible for several pathological conditions.

Oxidative damage depends on the co-existence of various factors: the intrinsic characteristics of the chemical structure of free radicals, the structural and functional properties of the molecular substratum which they contact, and finally the defensive antioxidant capacity of the body. Therefore, it is clear that any condition able to cause an alteration in the physiological balance between production and removal of free radicals and a deficit in the body's antioxidative defence mechanisms, results in the onset of a state of oxidative stress and the consequent outcome of pathological events.

Under physiological conditions and hence under conditions of adequate general wellbeing of the body, a precise balance exists between production and elimination of free radicals, associated with an adequate defense capability in the body, known as the antioxidant barrier.

The antioxidant barrier plays an absolutely preponderant role in the defensive action exerted by the body against free radical attacks. In fulfilling its antioxidant activity, it makes use of various molecules and enzyme systems of both exogenous origin, i.e. introduced into the body by means of either a suitable diet or synthetic preparations, and endogenous origin, i.e. produced directly by the cell's physiological metabolism.

These molecules having antioxidant action are able to neutralize free radicals, thus interrupting the radical chain propagation.

Natural enzyme systems having antioxidant activity are represented by superoxide-dismutase (SOD), which belongs to a family of metalloproteins whose function is to remove the superoxide anion radical (O₂); glutathione peroxidase (GPx) which has a reducing action on organic hydroperoxides using glutathione as co-substrate, and finally catalase (CAT) which reduces hydrogen peroxide (H₂O₂). Also involved in this defensive activity are vitamins, polyphenols, trace elements and various other substances which are taken through a suitable and correct diet. However, environmental pollution, cigarette smoke, incorrect diet, alcohol abuse, inadequate physical exercise, chronic consumption of medicines, and infections can all generate oxidative stress i.e. an imbalance between the production of reactive chemical species (free radicals) and the physiological capacity for defence (antioxidants). As aforementioned, oxidative stress leads to oxidative damage in nearly all the body's constituents and is therefore considered as being responsible for early ageing, as well as being associated with a series of very common diseases such as arterial hypertension, atherosclerosis, infarction, ictus, Parkinson's disease, Alzheimer's disease, obesity, diabetes, arthritis, some types of cancer, etc. In particular, oxidative stress appears to be the main cause of brain tissue damage in cases of prolonged hypoxia.

The object of the present invention is therefore to provide compounds able to act as antioxidants while at the same time being well tolerated by the body, having the lowest possible toxicity, as well as a high bioavailability.

### SUMMARY OF THE INVENTION

This object has been achieved by means of a compound of formula I: 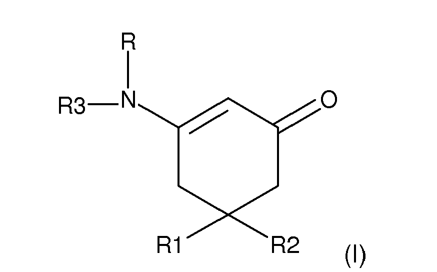 or a salt thereof, wherein
when R3 is 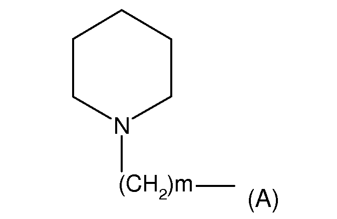 where m is 0, 2, or 3,
R is H or OH,
R1 is H or a C₁-C₃ alkyl group,
R2 is H, a C₁-C₃ alkyl group, phenyl or a substituted phenyl,
provided that when m is 0, R1 and R2 are not simultaneously CH₃, when R3 is 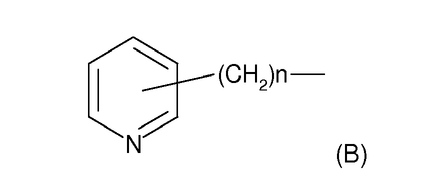 where n is 0, 1 or 2,
R is OH,
R1 is H or a C₁-C₃ alkyl group,
R2 is H, a C₁-C₃ alkyl group, phenyl or a substituted phenyl, when R3 is 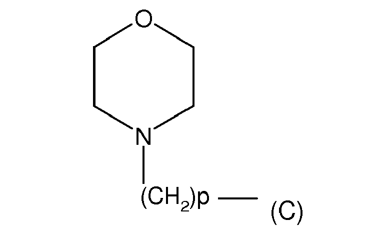 where p is 0, or 1,
R is H or OH,
R1 is H or a C₁-C₃ alkyl group,
R2 is H, a C₁-C₃ alkyl group, phenyl or a substituted phenyl,
provided that when p is 0, R1 and R2 are not simultaneously CH₃, are not simultaneously H, and are not simultaneously H and phenyl or a substituted phenyl.

In another aspect, the present invention relates to the preparation of said compounds.

In a further aspect, the present invention concerns the use of said compounds for treating cellular oxidative stress and in particular cerebral oxidative stress.

### BRIEF DESCRIPTION OF THE FIGURE

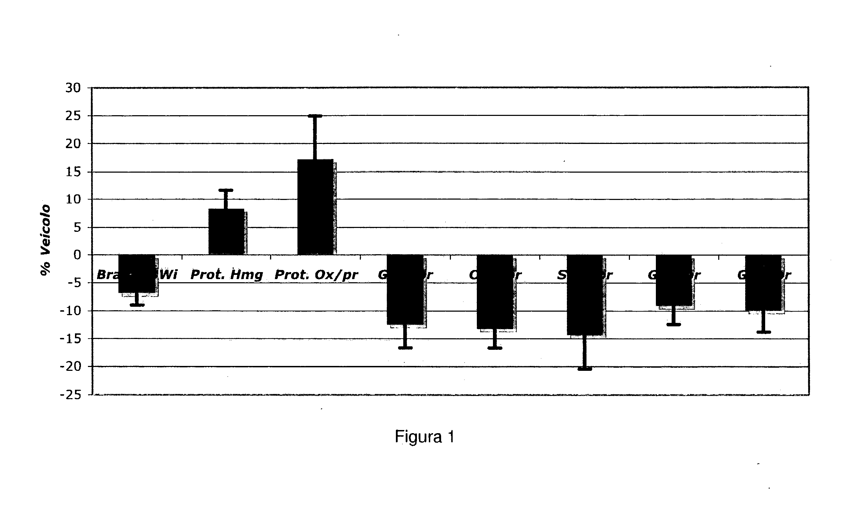

The characteristics and advantages of the present invention will be evident from the following detailed description, from the working examples provided for illustrative and non-limiting purposes, and from the annexed Figure 1 showing the detected parameters for doxorubicin-induced cerebral oxidative stress in mice.

### DETAILED DESCRIPTION OF THE INVENTION

The invention, therefore, concerns the compound of formula I: 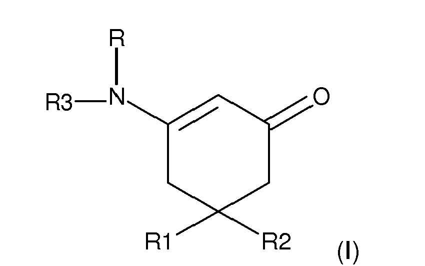 or a salt thereof, wherein
when R3 is 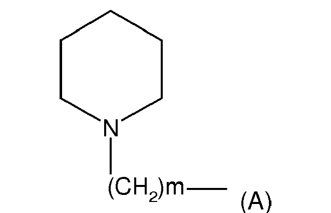 where m is 0, 2, or 3,
R is H or OH,
R1 is H or a C₁-C₃ alkyl group,
R2 is H, a C₁-C₃ alkyl group, phenyl or a substituted phenyl,
provided that when m is 0, R1 and R2 are not simultaneously CH₃, when R3 is 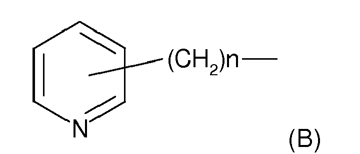 where n is 0, 1 or 2,
R is OH,
R1 is H or a C₁-C₃ alkyl group,
R2 is H, a C₁-C₃ alkyl group, phenyl or a substituted phenyl, when R3 is 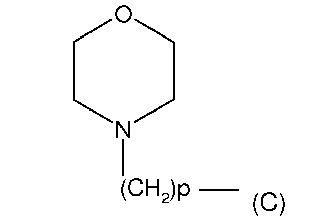 where p is 0, or 1,
R is H or OH,
R1 is H or a C₁-C₃ alkyl group,
R2 is H, a C₁-C₃ alkyl group, phenyl or a substituted phenyl,
provided that when p is 0, R1 and R2 are not simultaneously CH₃, are not simultaneously H, and are not simultaneously H and phenyl or a substituted phenyl.

According to the present invention, the term "substituted phenyl" means a phenyl substituted by at least one substituent group chosen from C₁-C₃ alkyl, halogen, - OY₁ and -NY₁Y₂, where Y₁ and Y₂ are, independently of each other, H or C₁-C₃ alkyl.

Preferably, when the compound of formula (I) is in the form of a salt, said salt is hydrochloride.

According to a preferred embodiment, the present invention relates to a compound of formula (I) 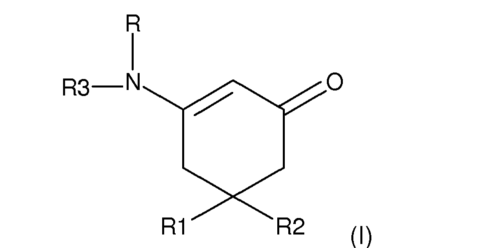 or a salt thereof, wherein
when R3 is 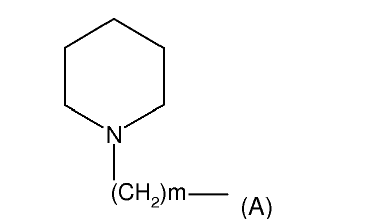 where m is 0, 2, or 3,
R is H or OH,
R1 is H or a C₁-C₃ alkyl group,
R2 is H, a C₁-C₃ alkyl group, phenyl or a substituted phenyl,
provided that when m is 0, R1 and R2 are not simultaneously CH₃, when R3 is 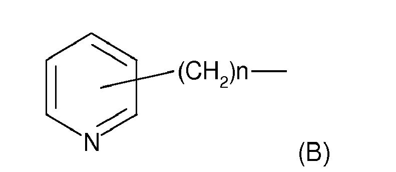 where n is 0, 1 or 2,
R is OH,
R1 is H or a C₁-C₃ alkyl group,
R2 is H, a C₁-C₃ alkyl group, phenyl or a substituted phenyl.

According to a more preferred embodiment, the present invention relates to a compound of formula (I)(A): 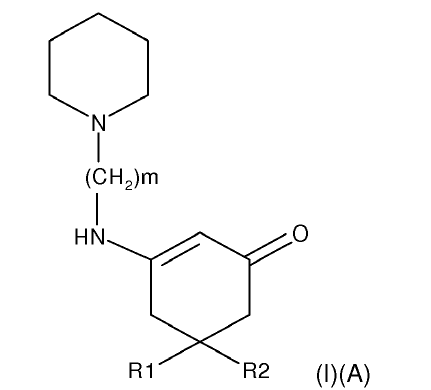 wherein
m is 0 or 2
R1 is H or a C₁-C₃ alkyl group, and
R2 is H, or a C₁-C₃ alkyl group, phenyl or a substituted phenyl.

Within this more preferred embodiment, the following compounds are further preferred:
(1) 5,5-dimethyl-3-(2-(piperidin-1-yl)ethylamino)cyclohex-2-enone 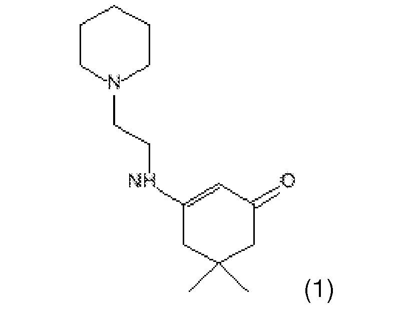
(2) 5-phenyl-3-(piperidin-1-ylamino)cyclohex-2-enone 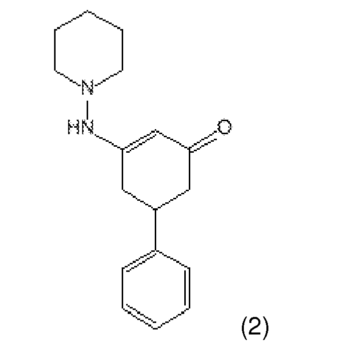
(3) 5-phenyl-3-(2-(piperidin-1-yl)ethylamino)cyclohex-2-enone

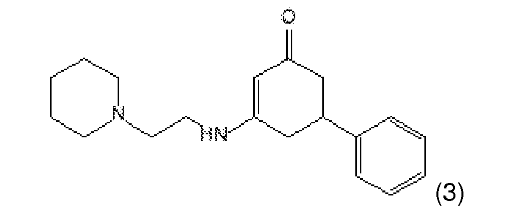

According to another more preferred embodiment, the present invention relates to a compound of formula (I)(B): 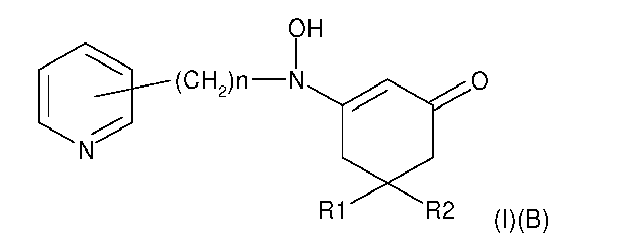 wherein
n is 0, 1 or 2,
R1 and R2 are, independently of each other, H or a C₁-C₃ alkyl group. Within said another more preferred embodiment, the following compounds are further preferred:
(4) 3-(hydroxy(pyridin-3-ylmethyl)amino)-5,5-dimethylcyclohex-2-enone 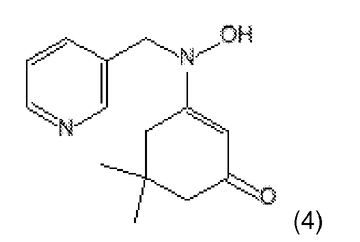
(5) 3-(hydroxy(pyridin-2-ylmethyl)amino)-5,5-dimethylcyclohex-2-enone

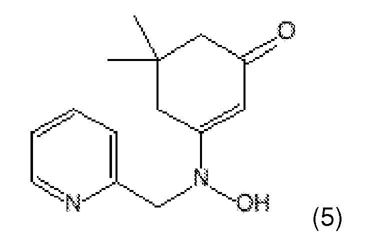

In another aspect, the present invention concerns a process for preparing the amino derivatives of cyclohex-2-enone of formula (I) as above described, comprising the step of reacting a compound (i) of formula 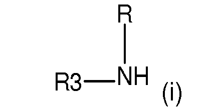 wherein
when R3 is 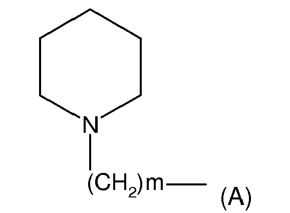 where m is 0, 2 or 3,
R is H or OH,
when R3 is 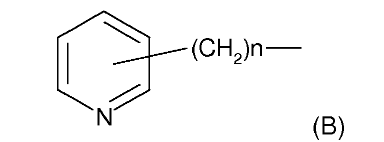 where n is 0, 1 or 2,
R is OH, when R3 is 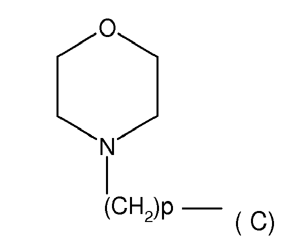 where p is 0, or 1,
R is H or OH,
and a compound (ii) of formula 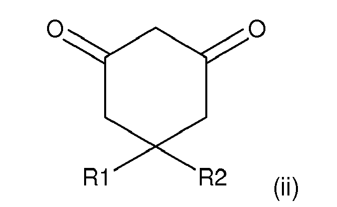 wherein
R1 is H or a C₁-C₃ alkyl group,
R2 is H a C₁-C₃ alkyl group, phenyl or a substituted phenyl.

Optionally, the amino derivative of cyclohex-2-enone compounds of formula (I) thus obtained are further salified. Preferably, when the compound of formula (I) is further salified, said compound is obtained in hydrochloride form.

According to a preferred embodiment, said process comprises the steps of:
- reacting the compound (i) and the compound (ii) in equimolar amounts in an alcoholic solution;
- heating to reflux for at least 15 minutes; and
- separating the compound of formula (I) thus obtained by solvent removal.

More preferably, said alcoholic solution comprises at least 50% of methanol. Optionally, the resulting compound of formula (I) is further ground with diethyl ether or with 2-propanol/hexane.

It is to be understood that all the aspects identified as preferred and advantageous for the compounds of formula (I) are accordingly considered to be preferred and advantageous for the preparation process of the present invention.

In another aspect, the present invention concerns a pharmaceutical antioxidant composition comprising at least one compound of formula (I) as above described and suitable pharmaceutical excipients. It has been surprisingly noted that the compounds of formula (I) not only perform a therapeutic antioxidant action, as will be fully demonstrated below, but also act as antioxidants for the pharmaceutical excipients of the composition itself, thus advantageously supporting the preservation of the same.

The pharmaceutical antioxidant composition according to the present invention can be administered orally or parenterally. In particular, said pharmaceutical antioxidant composition can be administered orally, intravenously, intramuscularly, intra-arterially, intramedullarily, intradurally, intracardially, transdermally, subcutaneously, intraperitoneally, intranasaly, intrarectally, sublingually or topically. The pharmaceutical antioxidant composition of the present invention can be prepared by known methods in the pharmaceutical art. In this respect, for oral administration the compounds of formula (I) can for example be mixed with an inert diluent and/or an edible carrier, enclosed within gelatin capsules or compressed in tablets. Otherwise, other compositions for injection can be prepared in the case of parenteral administrations by mixing the compounds of formula (I) with suitable diluents and/or excipients.

In another aspect, the invention concerns a cosmetic antioxidant composition comprising at least one compound of formula (I) as above described and suitable cosmetic excipients. Even in this case, the composition can be prepared in solid or liquid form, by mixing the compound of formula (I) with base ingredients, adjuvants, and/or additives commonly used in the cosmetic field. Among those, for instance water, alcohol, propylene glycol, stearic acid, glycerol, cetyl alcohol, and liquid paraffin can be used.

Cosmetic products comprising said composition can be creams, emulsions, lotions, gels, oils, beauty masks, foundation (liquids, pastes, powders), talc, soaps, bath and shower preparations, hair treatment products, sunscreen products, self-tanning products, skin-lightening products and anti-wrinkle products. It should be noted that the compounds of formula (I) also have an antioxidant effect on the cosmetic ingredients of the composition, thus advantageously supporting the preservation of the final cosmetic product itself.

In another aspect, the invention also concerns a food antioxidant composition comprising at least one compound of formula (I) as above described and suitable food ingredients. Said composition exhibits excellent antioxidant properties, which allow it to prevent deterioration of the food's aromas and fragrances, rancidification of fats, as well as food decolourization due to oxidation of its components. Therefore, the use of the food antioxidant composition of the invention in commonly consumed foodstuffs advantageously allows the conservation and freshness and hence the general quality thereof to be extended over time. Moreover, consumption of foods containing the food antioxidant composition of the invention enables the levels of oxidizing free radicals in the body to be reduced.

In a further aspect, the present invention concerns a compound of formula (I) 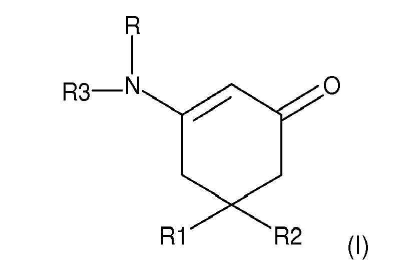 or a salt thereof, wherein
when R3 is 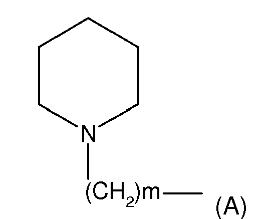 where m is 0, 2, or 3,
R is H or OH,
R1 is H or a C₁-C₃ alkyl group,
R2 is H, a C₁-C₃ alkyl group, phenyl or a substituted phenyl, when R3 is 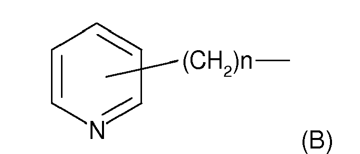 where n is 0, 1 or 2,
R is OH,
R1 is H or a C₁-C₃ alkyl group,
R2 is H, a C₁-C₃ alkyl group, phenyl or a substituted phenyl, when R3 is 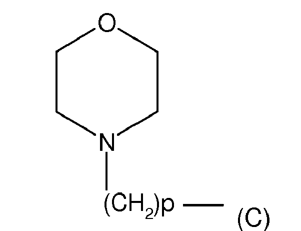 where p is 0, or 1,
R is H or OH,
R1 is H or a C₁-C₃ alkyl group,
R2 is H, a C₁-C₃ alkyl group, phenyl or a substituted phenyl,
provided that when p is 0, R1 and R2 are not simultaneously H, for use as a medicament.

Within said formula (I), the following compounds are further preferred:
(6) 5,5-dimethyl-3-(piperidin-1-ylamino)cyclohex-2-enone 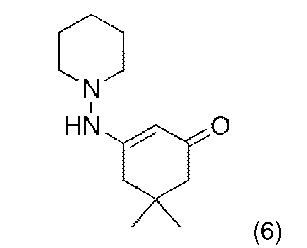
(7) 5,5-dimethyl-3-(morpholinoamino)cyclohex-2-enone

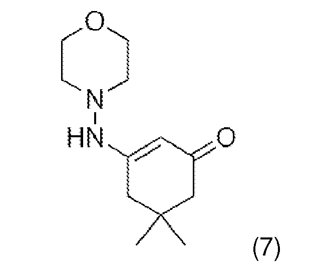

It is to be understood that all the aspects identified as preferred and advantageous for the compounds of formula (I) are accordingly considered to be preferred and advantageous for the use according to the present invention.

In particular, the present invention concerns a compound of formula (I) for treating cellular oxidative stress.

More preferably, said compound of formula (I) is used for treating cerebral cellular oxidative stress.

Indeed, as will be evident from the examples given below, it has been surprisingly found that the compounds of formula (I) of the invention are able not only to counteract free radical proliferation, but also to inhibit their formation, thus advantageously showing high antioxidant activity.

As a matter of fact, it should be reminded that, when maintained under homeostatic conditions, free radicals in tissues are necessary for correct cellular functioning, act as signaling molecules and as intermediates in enzymatic and chemical reactions; nevertheless transient states, wherein an increased presence of free radicals occurs, can also constitute fundamental physiological mechanisms. However, a chronic imbalance leading to greater tissue oxidation and hence a persistent oxidative stress, results in the onset of several diseases, even serious, such as cardiovascular and neurodegenerative diseases, tumours, immune and metabolic diseases as well as the acceleration of cellular ageing processes. In this situation, the compounds of the invention, through the many molecular mechanisms that intervene at various levels in the development of oxidative stress, can proved to be useful in treating the different pathologies ascribable to this complex event. Preferably, therefore, the present invention concerns the use of the compound of formula (I) for preparing a medicament for the treatment of cellular oxidative stress in diseases chosen from vascular diseases, cardiac diseases, tumours, neurodegenerative diseases, complications of ischemic events, imbalances of the immune system and metabolic diseases. More preferably, said pathologies are atherosclerosis, myocardial infarct, tumours dependent on oxidative damage to DNA, Alzheimer's disease, Parkinson's disease, Huntington's Chorea, amyotrophic lateral sclerosis, cerebral ischemia, immune system imbalances, diabetes, cerebral cell damage, multiple sclerosis, cerebral edema, ictus, consequences of cerebrovascular accident, cognitive deterioration, cerebral trauma, post-operative confusion, or epilepsy.

With reference to Example 8, the compounds of the invention were tested according to the TEAC and DPPH methods, which have enabled their ability to intercept free radicals to be observed. In view of the results obtained by said tests, the following compounds of the invention have been shown to be particularly advantageous as antioxidants and are, therefore, preferred:
(2) 5-phenyl-3-(piperidin-1-ylamino)cyclohex-2-enone
(6) 5,5-dimethyl-3-(piperidin-1-ylamino)cyclohex-2-enone
(7) 5,5-dimethyl-3-(morpholinoamino)cyclohex-2-enone

Most preferred is the compound:
(2) 5-phenyl-3-(piperidin-1-ylamino)cyclohex-2-enone

Additionally, tests were carried out both in vitro and in vivo to evaluate the bioavailability of the compounds of the invention at the target site, as widely reported in Examples 9-10.

From the results obtained by the tests performed in these examples, the following compounds of the invention have been shown to be particularly bioavailable, as well as able to cross the blood-brain barrier in effective amounts, and are thus preferred:
(5) 3-(hydroxy(pyridin-2-ylmethyl)amino)-5,5-dimethylcyclohex-2-enone
(1) 5,5-dimethyl-3-(2-(piperidin-1-yl)ethylamino)cyclohex-2-enone
(7) 5,5-dimethyl-3-(morpholinoamino)cyclohex-2-enone
(3) 5-phenyl-3-(2-(piperidin-1-yl)ethylamino)cyclohex-2-enone
(6) 5,5-dimethyl-3-(piperidin-1-ylamino)cyclohex-2-enone
(4) 3-(hydroxy(pyridin-3-ylmethyl)amino)-5,5-dimethylcyclohex-2-enone More preferred are the compounds:
(5) 3-(hydroxy(pyridin-2-ylmethyl)amino)-5,5-dimethylcyclohex-2-enone
(1) 5,5-dimethyl-3-(2-(piperidin-1-yl)ethylamino)cyclohex-2-enone
(7) 5,5-dimethyl-3-(morpholinoamino)cyclohex-2-enone

From the viewpoint of free radical formation inhibition, and with reference to Example 11, the preferred compounds are:
(1) 5,5-dimethyl-3-(2-(piperidin-1-yl)ethylamino)cyclohex-2-enone (3) 5-phenyl-3-(2-(piperidin-1-yl)ethylamino)cyclohex-2-enone With regard to toxicity evaluation, the compounds of the invention were tested in vivo, as reported in Example 13. In particular, the following compounds of the invention have proved to be advantageously well tolerated: (1), (4), (5), (6) and (7).

On the basis of all the tests undertaken, the following compounds are regarded as being preferred from the viewpoint of the best combination of results obtained:
(1) 5,5-dimethyl-3-(2-(piperidin-1-yl)ethylamino)cyclohex-2-enone
(7) 5,5-dimethyl-3-(morpholinoamino)cyclohex-2-enone
(6) 5,5-dimethyl-3-(piperidin-1-ylamino)cyclohex-2-enone
(5) 3-(hydroxy(pyridin-2-ylmethyl)amino)-5,5-dimethylcyclohex-2-enone

The following compounds are more preferred:
(1) 5,5-dimethyl-3-(2-(piperidin-1-yl)ethylamino)cyclohex-2-enone
(7) 5,5-dimethyl-3-(morpholinoamino)cyclohex-2-enone
Working examples of the present invention are hereinafter provided for illustrative and non-limiting purposes, together with examples of evaluating the antioxidative capacity of the thus prepared compounds and examples, both in vitro and in vivo, of their bioavailability.

### EXAMPLES

The following Examples 1-13 give the synthesis and characterization of the compounds of formula (I) according to the invention, as well as tests of antioxidant activity.

### Apparatus and materials

The following equipment was used during the implementation of all the examples:
- for melting point determination a Buchi B-540 instrument was used;
- for pH determination at the semi-neutralization point of titration (pKa) a Mettler Toledo DL-50 instrument was used with a 0.1 N xNaOH or HCl solution;
- the ¹H-NMR spectra were recorded at 300 MHz and the ¹³C-NMR spectra were recorded at 75 MHz using a Varian Unity 300 instrument;
- spectrophotometric measurements were carried out using a Lambda Perkin Elmer 15 UV/Vis spectrophotometer for the DPPH assay and a Wallac Victor 1420 multilabel counter spectrophotometer.

The following compounds were supplied by Aldrich:
- 2,2'-azino-bis(3-ethylbenzothiazoline-6-sulphonic acid) diammonium salt (ABTS)
- the free radical 2,2-diphenyl-1-picrylhydrazyl (DPPH);
- Horseradish peroxidase type VI-A (HRP),
- H₂O₂, citric acid, methanol, DMSO.

All the solutions of said compounds were prepared for carrying out the examples and used within 8 hours from their preparation.

### Example 1

### 5,5-dimethyl-3-(2-(piperidin-1-yl)ethylamino)cyclohex-2-enone

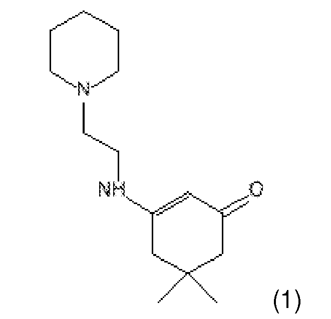

A mixture of 1-(2-aminoethyl)-piperidine (10 mmol), 5,5-dimethyl-1,3-cyclohexanedione (10 mmol) and methanol (15 ml) was heated to reflux for 20 minutes. The solvent was removed under reduced pressure (water bath temperature 50°C) to give the compound (1).
Yield: 68% after grinding with diethyl ether
Formula: C₁₅H₂₆N₂O
MW: 250.4 g/mol
Acid dissociation constants: pKa₁ = 2.82, pKa₂ = 8.64
m.p.: 128-129ºC
¹H-NMR (CDCl₃): δ 1.02 (6H, s), 1.41 (2H, m), 1.52 (4H, m), 2.13(2H, s), 2.16 (2H, s), 2.32 (4H, s), 2.48 (2H, t, J=5.5 Hz), 3.05 (2H, q, J=5.5 Hz), 5.02 (1 H, s), 5.28 (1 H, s, broad).
¹³C-NMR (CDCl₃): δ 24.9, 26.6, 29.0, 33.4, 39.6, 44.3, 51.1, 54.8, 56.6, 96.5, 163.0,197.2.

### Example 2

### 5-phenyl-3-(piperidin-1-ylamino)cyclohex-2-enone

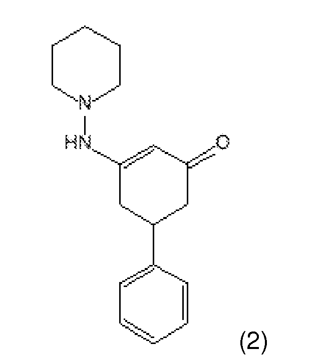

A mixture of N-aminopiperidine (10 mmol), 5-phenyl-1,3-cyclohexanedione (10 mmol) and methanol (15 ml) was heated to reflux for 20 minutes. The solvent was removed under reduced pressure (water bath temperature 50°C) to give the compound (2).
Yield: 88% after grinding with diethyl ether
Formula: C₁₇H₂₂N₂O
MW: 270.4 g/mol
m.p.: 240°C (dec).
¹H-NMR (CDCl₃): δ 1.38 (2H, s, broad), 1.63 (4H, m), 2.28-2.70 (8H, m, overlapping) 3.31 (1 H, m), 5.59 (1 H, s, broad), 5.74 (1 H, s, broad), 7.19-7.33 (5H, m).
¹³C-NMR (CDCl₃): δ 23.9, 26.2, 35.4, 40.7, 44.6, 57.1, 98.0, 127.4, 127.6, 129.4, 143.9, 162.2, 197.4.

### Example 3

### 5-phenyl-3-(2-(piperidin-1-yl)ethylamino)cyclohex-2-enone

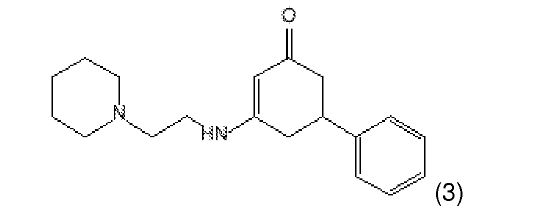

A mixture of 1-(2-aminoethyl)-piperidine (10 mmol), 5-phenyl-1,3-cyclohexanedione (10 mmol) and methanol (15 ml) was heated to reflux for 20 minutes. The solvent was removed under reduced pressure (water bath temperature 50°C) to give the compound (3).
Yield: 72% after grinding with diethyl ether
Formula: C₁₉H₂₆N₂O
MW: 298.4 g/mol
Acid dissociation constants: pKa₁ = 2.82, pKa₂ = 8.46
m.p.: 170.5-171.5 °C
¹H-NMR (CDCl₃): δ 1.42 (2H, m), 1.52 (4H, m), 2.30-2.81 (10H, m, overlapping), 3.11 (2H, m), 3.32 (1 H, m), 5.14 (1 H, s), 5.45 (1 H, s, broad), 7.18-7.34 (5H, m). ¹³C-NMR (CDCl₃): δ 24.9, 26.5, 38.0, 39.5, 40.7, 44.2, 54.7, 56.4, 97.3, 127.4, 127.6, 129.3, 144.0, 163.8, 197.0

### Example 4

### 3-(hydroxy(pyridin-3-ylmethyl)amino)-5,5-dimethylcyclohex-2-enone

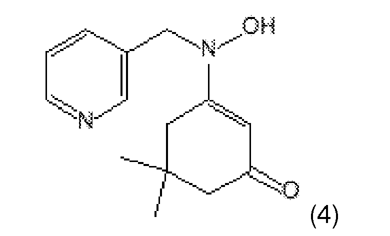

A mixture of (3-hydroxyaminomethyl)-pyridine (10 mmol), 5,5-dimethyl-1,3-cyclohexanedione (10 mmol) and methanol (15 ml) was heated at reflux for 20 minutes. The solvent was removed under reduced pressure (water bath temperature 50°C) to give the compound (4).
Yield: 58% after recrystallization from 2-propanol/hexane
Formula: C₁₄H₁₈N₂O₂
MW: 246.3 g/mol
Acid dissociation constants: pKa₁ = 2.70, pKa₂ = 6.47
m.p.: 169-170°C
¹H-NMR (MeOH-d₄): δ 1.06 (6H, s), 2.15 (2H, s), 2.36 (2H, s), 4.41 (2H, s), 5.02 (1 H, s), 7.42 (1 H, dd, J=5 Hz and 8 Hz), 7.78 (1 H, d, broad, J=8 Hz), 7.81 (1 H, t, J=8 Hz), 8.45 (1 H, d, J=5 Hz), 8.50 (1 H, s, broad).
¹³C-NMR (MeOH-d₄): δ 28.3, 33.7, 44.9, 48.2, 50.6, 95.5, 125.4, 135.3, 137.4, 149.2, 149.3, 167.8, 199.5

### Example 5

### 3-(hydroxy(pyridin-2-ylmethyl)amino)-5,5-dimethylcyclohex-2-enone

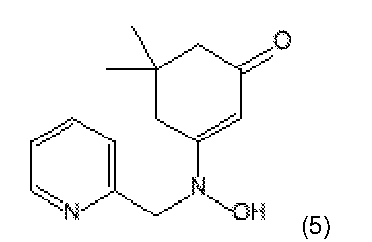

A mixture of (2-hydroxyaminomethyl)-pyridine (10 mmol), 5,5-dimethyl-1,3-cyclohexanedione (10 mmol) and methanol (15 ml) was heated at reflux for 20 minutes. The solvent was removed under reduced pressure (water bath temperature 50°C) to give the compound (5).
Yield: 65% after recrystallization from 2-propanol/hexane
Formula: C₁₄H₁₈N₂O₂
MW: 246.3 g/mol
Acid dissociation constant: pKa = 4.20
m.p.: 120-121 °C
¹H-NMR (MeOH-d₄): δ 1.06 (6H, s), 2.15 (2H, s), 2.40 (2H, s), 4.45 (2H, s), 5.02 (1 H, s), 7.32 (1 H, dd, J=5 Hz, J=8 Hz), 7.37 (1 H, d, J=8 Hz), 7.81 (1 H, t, J=8 Hz), 8.51 (1 H, d, J=5 Hz).
¹³C-NMR (MeOH-d₄): δ 28.3, 33.7, 43.6, 48.8, 50.6, 95.6, 123.0, 124.1, 138.9, 150.2, 158.0, 167.9, 199.4

### Example 6

### 5,5-dimethyl-3-(piperidin-1-ylamino)cyclohex-2-enone

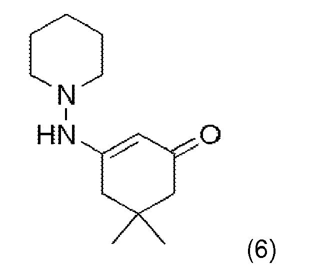

A mixture of N-aminopiperidine (10 mmol), 5,5-dimethyl-1,3-cyclohexanedione (10 mmol) and methanol (15 ml) was heated at reflux for 20 minutes. The solvent was removed under reduced pressure (water bath temperature 50°C) to give the compound (6).
Yield: 90% after grinding with diethyl ether
Formula: C₁₃H₂₂N₂O
MW : 222.3 g/mol
Acid dissociation constants: pKa = 2.62
m.p.: 189-190°C
¹H-NMR (CDCl₃): δ 1.04 (6H, s), 1.38 (2H, bs), 1.62 (4H, m), 2.08 (2H, s, broad), 2.17(2H, s), 2.64 (4H, s, broad), 5.64 (1 H, s), 5.76 (1 H, s, broad).
¹³C-NMR (CDCl₃): δ 23.9, 26.2, 28.9, 33.6, 41.5, 51.3, 56.9, 96.4, 162.0, 197.7

### Example 7

### 5,5-dimethyl-3-(morpholinoamino)cyclohex-2-enone

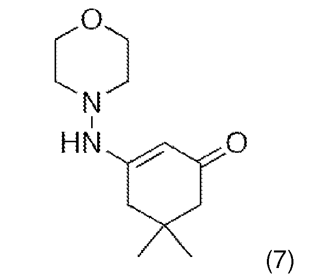

A mixture of 4-aminomorpholine (10 mmol), 5,5-dimethyl-1,3-cyclohexanedione (10 mmol) and methanol (15 ml) was heated at reflux for 20 minutes. The solvent was removed under reduced pressure (water bath temperature 50°C) to give the compound (7).
Yield: 88% after grinding with diethyl ether
Formula: C₁₂H₂₀N₂O₂
MW: 224.3 g/mol
Acid dissociation constant: pKa = 2.94
m.p.: 193-194 °C
¹H-NMR (CDCl₃): δ 0.98 (6H, s), 2.09 (4H, s), 2.70 (4H, s, broad), 3.65 (4H, s, broad), 5.57 (1 H, s, broad), 7.06 (1 H, s, broad).
¹³C-NMR (CDCl₃): δ 28.8, 33.5, 41.1, 51.3, 55.5, 67.1, 96.0, 162.5, 197.8

### Example 8

### Evaluation of the antioxidant activity of the compounds of the invention TEAC method (Trolox Equivalent Antioxidant Capacity Assay)

This assay is used to measure the total antioxidant activity of pure substances, biological fluids and plant compounds and is based on the reaction of antioxidants with the cation radical ABTS^{˙+} (2,2'-azinobis(3-ethylbenzothiazolin-6-sulphonate)). This cation radical is metastable and has to be generated just before the experiment by reacting ABTS with hydrogen peroxide in the presence of HRP.

This radical can be reduced, with consequent loss of absorbance, by an antioxidant whose scavenging capacity can be measured spectrophotometrically at 650 nm. The test is carried out at 37ºC and the results are obtained from a comparison with Trolox (a synthetic antioxidant and hydrophilic analogue of vitamin E).

The ABTS solution S₁ was prepared as follows:
20 mg of ABTS were added to 50 mM of citric acid buffer (40 ml) at pH=4.5 and to the resulting solution there was added an aqueous solution of HRP (4.4 mg, 400 µl) and an aqueous solution of H₂O₂ (10 µl of a 30% solution diluted with 16.3 ml of water).

For each of the compounds (2), (6) and (7), as synthesized above, there was prepared a 600 µM solution in water, methanol or DMSO according to its solubility, as well as a Trolox solution as reference.

Each solution was diluted to produce 6 solutions each at a concentration between 25 µM and 300 µM. From each of the diluted and divided solutions, there were withdrawn 20 µl to which 200 µl of solution S₁ were then added.

The decrease in absorbance of each solution thus obtained was monitored at 650 nm until a stable value was reached (about 90 minutes).

For each set of 6 solutions and for the reference trolox solution, the absorption was given in graph form as a function of the amount of compound (nmol) in the solution. The gradient of the straight line obtained by linear interpolation was determined according to the least squares method. The TEAC value of each compound of the invention is given in the table below and is expressed as the ratio between the gradient of the resulting straight line and the gradient of the reference (trolox) straight line.

Table 1 also shows the values obtained for the following comparative compounds: edaravone and vitamin C.

**Table 1**

| **Compound** | **TEAC** |
|---|---|
| (2) | 1.67 |
| (6) | 1.2 |
| (7) | 0.8 |
| edaravone | 0.9 |
| vitamin C | 0.9 |

### DPPH method (2,2-diphenyl-1-picrylhydrazyl free radical)

DPPH (diphenylpicrylhydrazyl radical) is one of the most stable and easy-to-use free radicals; it is normally used to test the capacity of some compounds to act as free radical scavengers.

DPPH in solution is violet in colour with maximum absorbance at 515 nm varying from violet to yellow when this radical removes a hydrogen atom from a scavenger antioxidant to form the reduced form, DPPH-H. This reaction can therefore be easily monitored by spectrophotometry.

Based on the decrease in absorption at 515 nm, the different parameters that define the antioxidant strength itself can be calculated, including the EC₅₀ value, i.e. the amount of antioxidant needed to halve the initial DPPH radical concentration.

The solution S₁ of DPPH was prepared as follows:
DPPH (10.0 mg, 25.3 µmol) was dissolved in methanol (42.3 ml) and 10 ml of the resultant solution (600 µM) were added to the solution resulting from diluting citric acid buffer (10 mM pH=7.4) (42 ml) with methanol (62 ml).

Then 700 mM solutions of each compound (2), (6) and (7), as synthesized above, were prepared by dissolving in methanol. Each solution was diluted to produce 6 solutions each at a concentration between 70 and 70000 µM. From each of the diluted and divided solutions there were withdrawn 0.1 ml to which 3.9 ml of solution S₁ were then added.

The decrease in absorbance of each solution obtained in this manner was monitored at 515 nm until a stable value was reached (about 400 minutes).

A reference solution of DPPH (3.9 ml S₁ and 0.1 ml) was also monitored to evaluate DPPH decomposition during the measurement period. For each solution the decrease in absorbance was subtracted from the absorbance of the reference solution extrapolated at 400 minutes, and was shown as a graph over the logarithmic concentration of the compound, thus obtaining a straight line. From this latter, the concentration corresponding to the halving of absorbance (EC₅₀) was determined as shown in table 2.

**Table 2**

| **Compound** | **DPPH (EC₅₀)** |
|---|---|
| (2) | 0.72 |
| (6) | 1.3 |
| (7) | 12 |
| edaravone | 1.9 |
| vitamin C | 1.67 |

From the aforegiven results it can be seen that the tested compounds were positive in both assays, i.e. able to intercept free radicals and hence to have antioxidant activity. Indeed, said activity was found to be comparable for the most part with that of vitamin C and Trolox, which are well-known antioxidants.

### Example 9

### In vivo assay of cerebral oxidative stress induced in mice by doxorubicin

The assay was carried out on compounds (1) and (3)-(6) and on edaravone (E) as comparative compound.

10 mice were used for each treatment group. For each compound 50 mg/kg were administered intraperitoneally (IP) for three consecutive days (day 0, 1 and 2); half the animals (5) also received IP 16.7 mg/kg of doxorubicin immediately after the first treatment (day 0). On the day after the last administration (day 3) the animals were killed by neck dislocation and the brains were removed, weighed and frozen in liquid nitrogen.

For the analyses of oxidative stress, 100 mg/ml of brain homogenate was prepared in Hebe buffer (pH 7.4 EDTA/HEPES^{(**)}) containing a mixture of enzyme inhibitors (Leupeptin, Pepstatin, Aprotinin, BHA).
^{(*)} Hypotonic Extraction Buffer
^{(**)} 4-2-hydroxyethyl-1-piperazinyl-ethanesulphonic acid

The evaluation parameters were:
- BrW/BWi: ratio between final brain weight and initial body weight (day 0);
- Prot. Hmg: total proteins in the homogenate (BCA method, i.e. bicinchoninic acid);
- Prot. Ox: oxidized proteins in the Hmg by means of a colorimetric method using dinitrophenylhydrazone (DNPH);
- GSH: reduced glutathione in the supernatant (sn) of the Hmg, obtained after precipitation with metaphosphoric acid (MPA), by means of a colorimetric method using dithiodinitrobenzene (DTNB);
- CAT: catalase in the Hmg by a colorimetric method using potassium dichromate;
- SOD: superoxide dismutase by a colorimetric method using SOD Reagent (xanthine, EDTA, nitroblue tetrazole, Na₂CO₃, BSA, i.e. bovine serum albumin);
- GST: glutathione S-transferase in the supernatant by a colorimetric method using chlorodinitrobenzene (CDNB) and GSH; and
- GPx: glutathione peroxidase in the supernatant after addition of GSH and tert butyl hydroperoxide (t-BuOOH), heating and precipitation with MPA; followed by GSH method with DTNB.

### Model

### The mean effect (n=5 experiments) of doxorubicin (D), i.e. "oxidized" animal, relative to the vehicle (V), the vehicle representing the healthy animal.

All the parameters, with the exclusion of Brain/BWi and Prot. Hmg, were normalized for total Hmg proteins (Table 3). The D value standard deviations and the % incidence relative to V are given in italics.

**Table 3**

| **Group** | **Brain/BWi** | **Prot. Hmg** | **Prot. Ox** | **GSH** | **CAT** | **SOD** | **GST** | **GPX** |
|---|---|---|---|---|---|---|---|---|
| | (g/kg) | (mg/ml) | (µmol H₂O₂/mg) | (nmol/mg) | (U/mg) | (mU/mg) | (µg/mg) | (U/mg) |
| **V** | 19.6467 | 9.1027 | 2.3373 | 8.6427 | 2.2487 | 36.6067 | 20.4067 | 5.5853 |
| **D** | 18.2867 | 9.8424 | 2.7720 | 7.5167 | 1.9460 | 31.2667 | 18.5067 | 5.0010 |
| *St.Dv. D* | *0.3908* | *0.2997* | *0.1993* | *0.3612* | *0.0761* | *2.2715* | *0.6822* | *0.2193* |
| **%D** | 93.1 | 108.3 | 117.2 | 87.5 | 86.8 | 85.7 | 90.9 | 90.1 |
| *St.Dv %D* | *2.0* | *3.3* | *7.7* | *4.1* | *3.4* | *6.1* | *3.3* | *3.9* |
| **Diff. D-V** | -1.3600 | 0.7397 | 0.4347 | -1.1260 | -0.3027 | -5.3400 | -1.9000 | -0.5843 |

As can be seen from Table 5 and with reference to Figure 1, a "Doxo" effect was observed with all the evaluated parameters, varying from a 6.9% minimum for Brain/BW to a maximum of 17.2% for Prot. Ox. Bearing in mind that said animal model of cerebral oxidation is based on the generalized oxidative and degenerative effects caused by the systemic administration of doxorubicin, and consequently also the effects on the brain, these variations enabled a preliminary evaluation of those parameters acted upon by the compounds of the invention, as well as their potency.

### Effect of the compounds of the invention on healthy animals

The mean % effect, on all the parameters simultaneously, of the triple treatment in the healthy animals, as mean percentage variation relative to the vehicle (table 4). This value is useful as an approximate safety index of the oxidative state of the brain, i.e. the capacity of the compounds of the invention not to alter cerebral homeostasis in healthy animals.

**Table 4**

| | Mean % variation relative to the vehicle |
|---|---|
| **D** | **-11.4** |
| V+(6) | -5.4 |
| V+(1) | -4.3 |
| V+E | -2.7 |
| V+(3) | -1.3 |
| V+(5) | -0.4 |
| V+(4) | 0.0 |

As can be seen in Table 4, the tested compounds were found to be ideal since, like the comparative compound, on the whole they did not significantly alter the parameters of the "healthy" animals after the same treatment destined for the "oxidized" animals.

The details of the effect of the aforesaid compounds on the individual parameters are shown below:

**Table 5**

| **Group** | **Brain/BWi** | **Prot. Hmg** | **Prot. Ox/pr** | **GSH/pr** | **CAT/pr** | **SOD/pr** | **GST/pr** | **GPX/pr** |
|---|---|---|---|---|---|---|---|---|
| ***D*** | ***-6.9*** | ***8.3*** | ***17.2*** | ***-12.5*** | ***-13.2*** | ***-14.3*** | ***-9.1*** | ***-9.9*** |
| V+(1) | -3.2 | 5.3 | 1.4 | -1.5 | -7.1 | -5.9 | -2.3 | -7.6 |
| V+(3) | 1.0 | 2.3 | -7.8 | 0.4 | -9.2 | 1.1 | 4.1 | 2.4 |
| V+(4) | -0.1 | 1.2 | -10.3 | -0.2 | -9.3 | 0.0 | 0.5 | 0.1 |
| V+(5) | -3.3 | 0.7 | -5.5 | -0.9 | -6.2 | -2.3 | 3.1 | 1.5 |
| V+(6) | -2.5 | 8.4 | -5.6 | -9.6 | -6.4 | -10.8 | -2.5 | -8.7 |
| V+E | 2.7 | 3.7 | -2.3 | -5.5 | -5.0 | -2.2 | -3.1 | -6.7 |

### Effect of the compounds of the invention on the oxidized animals

The effect of the tested compounds on the "oxidized" animals was given as % inhibition relative to the percentage variation due to doxorubicin alone. The effect of the compounds on the oxidized animals can be expressed both as a function of the values of the **vehicle** (healthy, vehicle only) and as a function of the value of **its own** control (healthy, compound only):
- **Vehicle:** percentage [(Compound+D)/V]x100/[(V+D)/V]x100
- **Its own:** percentage [(Compound+D)/Compound]x100/[(V+D)/V]x100

Said percentage inhibition values, for all the analyzed parameters, are given in table 6 below.

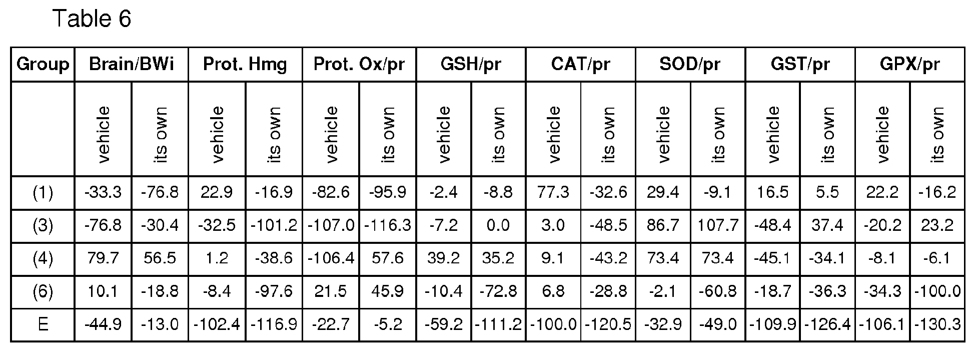

### Results

As can be seen from Table 6, both with respect to the Vehicle and Its own, the comparative compound had an overall significant inhibitory effect on the doxorubicin-induced oxidation (about 75%). With respect to Its own vehicle, the compounds (3), (6) and (1) also showed an overall percentage inhibition as being significantly greater than 40%. With regard to safety (Table 4), the compounds of the invention, like the comparison compound, presented values that complied with the regulations (± 5.5%).

### Example 10

### Brain-to-plasma concentration ratio of the compounds of the invention in mice

The ability of the compounds of the invention to penetrate the brain was investigated by measuring their brain/plasma (B/P) concentration ratio in mice following intraperitoneal administration. The B/P ratio of the compounds of the invention and the B/P ratio of caffeine and edaravone as comparative compounds, were also compared.

### Materials and methods

Female CD-1 mice were used (n=3 for each time period) and received the compounds of the invention by intraperitoneal administration, in 25 mg/kg doses in a 0.5% NaHCO₃ buffer (pH 8.3). At predefined intervals following said administration, i.e. 5, 15, 30 and 60 minutes, the mice were killed and both plasma and total brain tissue were collected.

Specifically, after anaesthesia with ether, the animals were bled by blood withdrawal from the abdominal aorta then immediately decapitated. The blood samples were collected into plasma separator tubes containing Li-Heparin as anticoagulant and centrifuged. The resulting plasma was then stored at -20ºC. The brain was rapidly removed from the cranium and weighed, subsequently frozen and stored at -20ºC before treatment by homogenization and HPLC analysis.

The plasma was thawed and mixed with an equal volume of the extraction solution. This extraction solution was a mixture of aqueous and organic solvents whose composition had been previously determined by HPLC undertaken for each analyte.

1 ml of solution was then extracted by adding 20 µl of 70% HClO₄ then centrifuged at 15,500g for 5 minutes and injected into the HPLC.

Each brain was thawed and homogenized with the extraction solution to provide a final composition of 4 ml/1g of tissue by means of an Ultra-Turrax blender and sonicated for 5 minutes. After centrifugation of the homogenate at 15,500g for 5 minutes, the resulting solution was treated with HClO₄, as indicated for the plasma.

Specific chromatographic conditions had been developed for each analyte, the calibration curve being constructed for plasma and brain homogenate.

### Results

The compounds (1), (3)-(7) presented a B/P ratio greater than 0.100, calculated by means of the area under the curve of concentrations in brain and in plasma over time (from 0 to 60 minutes). This value was considered ideal in view of the fact that the reference compound (edaravone), regarded as a compound which easily penetrates the blood-brain barrier, presented a value of 0.123. Many of the compounds of the invention advantageously presented values up to 5 times greater than edaravone, i.e. half that of caffeine i.e. 1.011, which, with regard to cerebral bioavailability, is the ideal compound. In this respect, a value of 1 indicates a complete cerebral bioavailability, which occurs when what is in plasma is likewise present in the brain.

### Example 11

### Evaluation of in vitro inhibition of CHP-induced oxidative stress by the compounds of the invention in C6 cells (rat glioma) and BAEC cells (bovine aortic endothelial)

It was investigated whether and to what extent the compounds of the invention (1 )-(7) were able to inhibit formation of cellular radicals and, hence, generation of oxidative stress in cells induced by CHP, i.e. cumene hydroperoxide, by employing dihydro-2',7'-dichlorofluorescein diacetate (H₂DCF-DA, Sigma Aldrich) as fluorescent probe able to react with reactive oxygen species.

### Materials and methods

The method used was essentially the one reported by Rüweler M. et al. ("Inhibition of peroxide-induced radical generation by plant polyphenols in C6 astroglioma cells" (2008) Toxicol in vitro 22, 1377-81), with a few minor modifications.

The cells were seeded in phenol-red free DMEM (Sigma Aldrich) into 96-well plates with 5% FBS and 20 mM HEPES at a density of 40,000 cells/well. After 48 hours, the medium was substituted by HBSS (Hanks' Balanced Salt Solution, Sigma) containing 10 µM of dihydro-2',7'-dichlorofluorescein diacetate (H₂DCF-DA, Sigma Aldrich) and incubated in darkness for 30 minutes at 37ºC, in order to load the cells with fluorescent dye. H₂DCF-DA was hydrolyzed enzymatically by the intracellular esterases to form non-fluorescent H₂DCF, which was then rapidly oxidized to form 2',7'-dichlorofluorescein (DCF) being highly fluorescent in the presence of reactive species (RS). The intensity of DCF fluorescence is directly proportional to the amount of RS formed.

After carrying out two washes, the cells were exposed to 500 µM of CHP and various concentrations (4, 20, 100 and 500 µM) of the compounds of the invention for 30 minutes at 37ºC. Fluorescence was then measured, after cell washing, by a microplate reader at an excitation wavelength of 485 nm and an emission wavelength of 535 nm.

Six wells per plate per concentration of test composition (compound + CHP) and per control (HBSS without CHP) were measured and 12 wells were used as reference for CHP-induced stress. In addition, each 96-well plate contained 6 wells that received the highest concentration of the tested compounds without CHP, to investigate whether the compounds of the invention alone were able to induce cellular radical formation.

When using this assay, it should be borne in mind that DCF fluorescence is a generalized indicator of oxidative stress rather than of any particular reactive species, i.e. it is not a direct assay for H₂O₂ or other reactive species (Halliwell B, Whiteman M. (2004) "Measuring reactive species and oxidative damage in vivo and in cell culture: how should you do it and what do the results mean?" Br. J. Pharmacol. 142 231-255). Therefore, when an increase in DCF fluorescence induced by exposure of cells to CHP was measured, as was undertaken in these assays, it was not possible to deduce which reactive species was responsible. Inhibition of CHP-induced fluorescence was evaluated by means of different concentrations of the compounds of the invention and comparative compounds such as Trolox and edaravone.

### Results

Granted that none of the tested compounds influenced the level of DCF fluorescence (same values for the control, data not shown), the compounds (1) and (3) were found to be effective since they presented inhibition values that were advantageously within the order of magnitude of the reference substances, Trolox and edaravone (10-50% depending on the concentrations and cell line).

### Example 12

### Genotoxicity tests: in vitro assay on mouse lymphoma undertaken for compounds (1), (3)-(7)

The assay utilized a clone of the murine lymphoma cell line designated as L5178Y. This clone has the characteristic of having two different chromosomes number 11, where the gene for thymidine kinase (tk) in the mouse is mapped:
- the chromosome designated 11 a presents a terminal deletion on the short arm, while
- the chromosome designated 11 b appears longer.

This morphological difference also reflects a genetic difference as the tk gene is the most distal on the short arm and hence only chromosome 11 b carries a copy of the functional gene.

The genetic characteristics of the clone, namely L5178Y tk^{+/-} 3.7.2C, are such that a mutational event to the only functional copy of the tk gene, this being an enzyme involved in the so-called salvage biosynthesis of pyrimidine nucleotides, renders the cell resistant to the pyrimidine analogue trifluorothymidine.

The relevant characteristic of the assay is that a functional mutation on the tk gene will give a mutant clone of different dimensions according to the type of event:
- point mutation = the clone, though mutant, maintains its genetic fitness, a nearly normal cell cycle and a dimension defined as "large" i.e. > ¼ of the well area in a 96-well plate,
- clastogene damage = in this case, in addition to the tk gene, other genes are also involved which alter the cell cycle and slow it down; therefore the mutant clone will have a "small" phenotype, i.e. ≤ ¼ of the well area.

This characteristic is the major advantage of the MLA (Mouse Lymphoma Assay) compared with other in vitro genotoxicity assays: one assay alone enables information to be obtained both on the mutagenic potency and clastogenic potency of a molecule over a short time, not requiring microscope analysis of the metaphases as a chromosome aberration test.

Advantageously, none of the compounds tested were found to have genotoxic activity neither in the presence nor absence of the metabolic activation system (S9)*.

^{(*)} The metabolic activation system (S9) also allows indirect mutagens to be detected, i.e. those substances which in order to become so must first be transformed by the body.

### Example 13

### Acute toxicity study on the compounds (1), (4)-(7) of the invention administered intraperitoneally in female CD-1 mice

This study was carried out with the purpose of investigating the toxicity of a single dose of the listed compounds administered to ICR (CD-1) female mice. According to the current OECD guideline no. 420, the entire study was divided into a preliminary study in which the effects of a dose of each compound were tested on a single animal for two weeks and, a main study, in which the dosage chosen for each compound based on observations undertaken in the preliminary study was administered to 5 mice which were observed for 14 days after treatment.

The mice used were all female and were treated by intraperitoneal injection administration. The animals were observed individually and regularly for the first 4 hours and within 24 hours after administration, and then twice a day for the whole observation period. As soon as the animals were stabilized, observations at the weekend were undertaken once only during the day.

The evaluated parameters included the survival, body weight development, variations in skin and fur, changes in ocular, conjunctival and buccal mucous membranes, changes in activities of the respiratory, circulatory, autonomous and central nervous systems, changes in somatomotor activity and in behaviour.

Any appearance of tremors, convulsions, salivation, diarrhea, lethargy, drowsiness and coma were attentively and regularly monitored. At the end of the observation period, a post-mortem was carried out on all the animals.

### Results

On the basis of the observed results, it was possible to identify for all the compounds a MTD (Maximum Tolerated Dose) comprised between 300 and 500 mg/kg pc, these being advantageously suitable tolerability values as well as fully conforming to legal requirements as regards new drug development.

From the detailed description and from the aforegiven examples, the advantages achieved by means of the compounds of the present invention are evident. In particular, said compounds have proved to be surprisingly and advantageously able to act as antioxidants while at the same time being well tolerated by the body, having the lowest possible toxicity, as well as high bioavailability.

## Claims

1. A compound of formula (I): 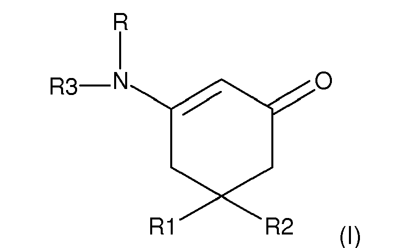 or a salt thereof, wherein
when R3 is 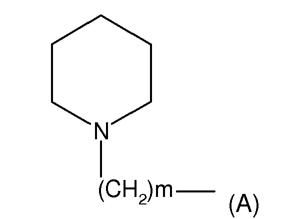 where m is 0, 2, or 3,
R is H or OH,
R1 is H or a C₁-C₃ alkyl group,
R2 is H, a C₁-C₃ alkyl group, phenyl or a substituted phenyl,
provided that when m is 0, R1 and R2 are not simultaneously CH₃, when R3 is 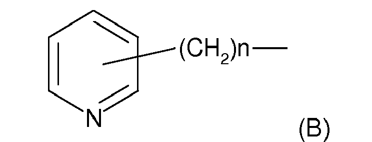 where n is 0, 1 or 2,
R is OH,
R1 is H or a C₁-C₃ alkyl group,
R2 is H, a C₁-C₃ alkyl group, phenyl or a substituted phenyl, when R3 is 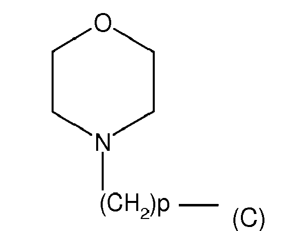 where p is 0, or 1,
R is H or OH,
R1 is H or a C₁-C₃ alkyl group,
R2 is H, a C₁-C₃ alkyl group, phenyl or a substituted phenyl,
provided that when p is 0, R1 and R2 are not simultaneously CH₃, are not simultaneously H, and are not simultaneously H and phenyl or a substituted phenyl.

2. The compound according to claim 1, of formula (I) 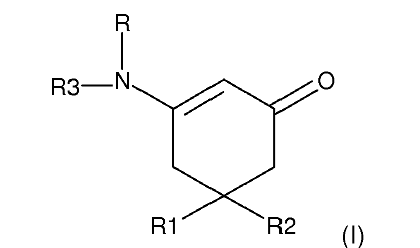 or a salt thereof, wherein
when R3 is 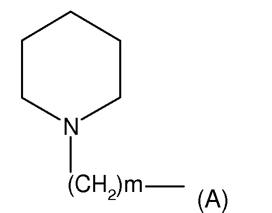 where m is 0, 2, or 3,
R is H or OH,
R1 is H or a C₁-C₃ alkyl group,
R2 is H, a C₁-C₃ alkyl group, phenyl or a substituted phenyl,
provided that when m is 0, R1 and R2 are not simultaneously CH₃, when R3 is 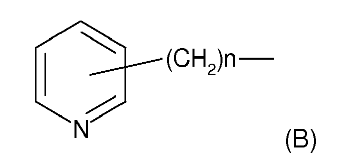 where n is 0, 1 or 2,
R is OH,
R1 is H or a C₁-C₃ alkyl group,
R2 is H, a C₁-C₃ alkyl group, phenyl or a substituted phenyl.

3. The compound according to claim 2, of formula (I)(A): 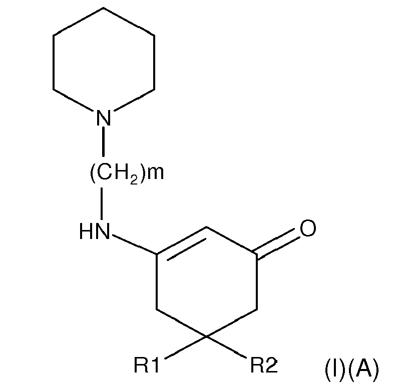 wherein
m is 0 or 2,
R1 is H or a C₁-C₃ alkyl group, and
R2 is H, or a C₁-C₃ alkyl group, phenyl or a substituted phenyl.

4. The compound according to claim 3, of formula (1)

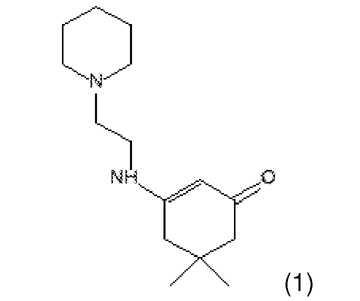

5. The compound according to claim 3, of formula (2)

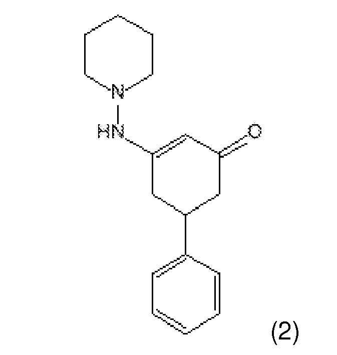

6. The compound according to claim 3, of formula (3)

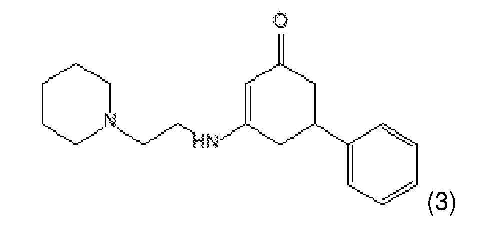

7. The compound according to claim 2, of formula (I)(B): 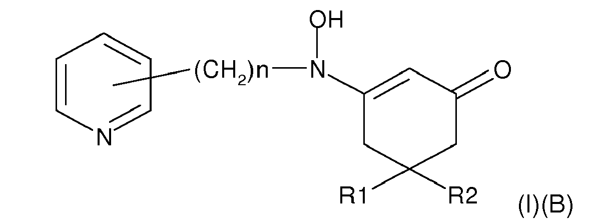 wherein
n is 0, 1 or 2,
R1 and R2 are, independently of each other, H or a C₁-C₃ alkyl group.

8. The compound according to claim 7, of formula (4)

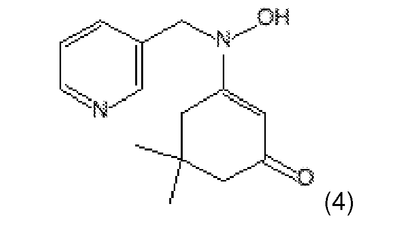

9. The compound according to claim 7, of formula (5)

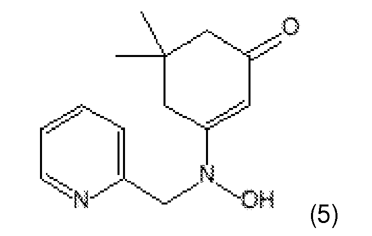

10. A process for preparing the compound of formula (I) of claim 1, comprising the step of reacting a compound (i) of formula 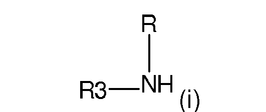 wherein
when R3 is 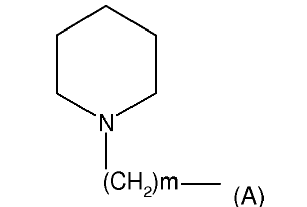 where m is 0, 2 or 3,
R is H or OH,
when R3 is 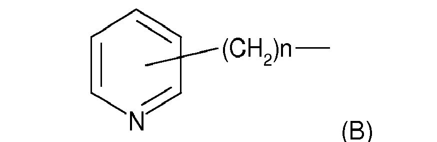 where n is 0, 1 or 2,
R is OH,
when R3 is 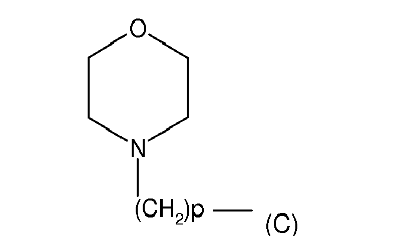 where p is 0, or 1,
R is H or OH,
and a compound (ii) of formula 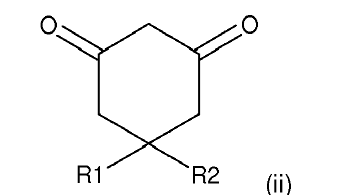 wherein
R1 is H or a C₁-C₃ alkyl group,
R2 is H, a C₁-C₃ alkyl group, phenyl or a substituted phenyl,
and, optionally, the step of salifying the compound of formula (I) thus obtained.

11. The process according to claim 10, wherein, when the compound of formula (I) is salified, said compound of formula (I) is obtained in a hydrochloride form.

12. A pharmaceutical antioxidant composition comprising at least one compound of formula (I) according to claim 1 and suitable pharmaceutical excipients.

13. A cosmetic antioxidant composition comprising at least one compound of formula (I) according to claim 1 and suitable cosmetic excipients.

14. A food antioxidant composition comprising at least one compound of formula (I) according to claim 1 and suitable food ingredients.

15. The compound of formula (I) 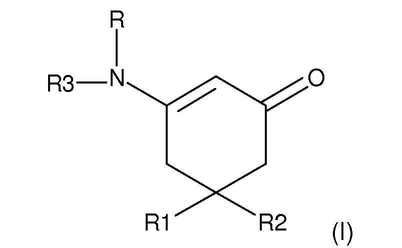 or a salt thereof, wherein
when R3 is 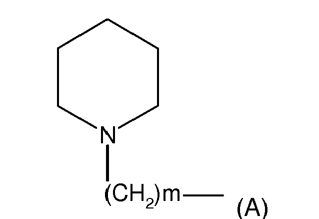 where m is 0, 2, or 3,
R is H or OH,
R1 is H or a C₁-C₃ alkyl group,
R2 is H, a C₁-C₃ alkyl group, phenyl or a substituted phenyl, when R3 is 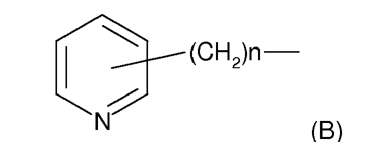 where n is 0, 1 or 2,
R is OH,
R1 is H or a C₁-C₃ alkyl group,
R2 is H, a C₁-C₃ alkyl group, phenyl or a substituted phenyl, when R3 is 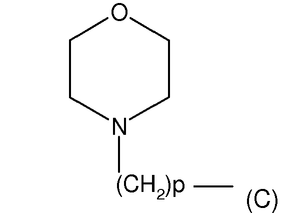 where p is 0, or 1,
R is H or OH,
R1 is H or a C₁-C₃ alkyl group,
R2 is H, a C₁-C₃ alkyl group, phenyl or a substituted phenyl,
provided that when p is 0, R1 and R2 are not simultaneously H, for use as a medicament.

16. The compound according to claim 15, of formula (6):

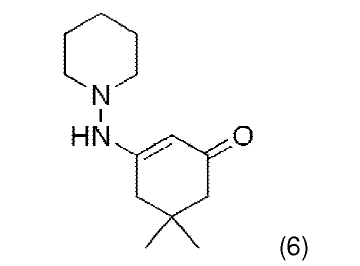

17. The compound according to claim 15, of formula (7):

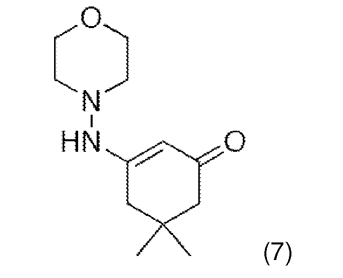

18. The compound according to claim 15, for the treatment of cellular oxidative stress.

19. The compound according to claim 18, for the treatment of cerebral cellular oxidative stress.

20. The compound according to claim 18, for the treatment of cellular oxidative stress in diseases selected from vascular diseases, cardiac diseases, tumours, neurodegenerative diseases, complications of ischemic events, imbalances of the immune system and metabolic diseases.

21. The compound according to claim 20, wherein said diseases are atherosclerosis, myocardial infarct, tumours dependent on oxidative damage to DNA, Alzheimer's disease, Parkinson's disease, Huntington's Chorea, amyotrophic lateral sclerosis, cerebral ischemia, imbalances of the immune system, diabetes, cerebral cell damage, multiple sclerosis, cerebral edema, ictus, consequences of cerebrovascular accident, cognitive deterioration, cerebral trauma, post-operative confusion, or epilepsy.
